# EUROPEAN PATENT SPECIFICATION

(11) **EP 3 292 102 B1**
(45) Date of publication and mention of the grant of the patent: **08.09.2021**
(21) Application number: 16728101.3
(22) Date of filing: 03.05.2016
(51) Int. Cl.: C07D 209/46

(54) **ISOINDOLINONES, PROCESSES FOR THE PRODUCTION OF CHIRAL DERIVATIVES THEREOF AND USE THEREOF**
ISOINDOLINONE, VERFAHREN ZUR HERSTELLUNG CHIRALER DERIVATE DAVON UND DEREN VERWENDUNG
ISOINDOLINONES, PROCÉDÉS DE PRODUCTION DE DÉRIVÉS CHIRAUX DE CELLES-CI ET UTILISATION DE CELLES-CI

(30) Priority: 05.05.2015 IT RM20150196
(43) Date of publication of application: 14.03.2018
(73) Proprietor: Universitá Degli Studi Di Salerno, 84084 Fisciano (SA) (IT)
(72) Inventor: MASSA, Antonio, 84084 Fisciano SA (IT); DI MOLA, Antonia, 84084 Fisciano SA (IT); DE CAPRARIS, Paolo, 84084 Fisciano SA (IT)
(74) Representative: Germinario, Claudio
(86) International application number: PCT/IB2016/052507
(87) International publication number: WO 2016/178140

(56) References cited:
- EP-A1- 0 529 778
- EP-A1- 1 566 378
- US-A- 4 244 966
- US-A- 6 087 364
- US-A1- 2013 261 103
- THOMAS R. BELLIOTTI ET AL: "Isoindolinone enantiomers having affinity for the dopamine D4 receptor", BIOORGANIC & MEDICINAL CHEMISTRY LETTERS, vol. 8, no. 12, June 1998 (1998-06), pages 1499-1502, XP055242530, AMSTERDAM, NL ISSN: 0960-894X, DOI: 10.1016/S0960-894X(98)00252-2 cited in the application
- ROMAIN SALLIO ET AL: "Enantioenriched Isoindolinones from Chiral Phase-Transfer-Catalyzed Intramolecular Aza-Michael Reactions", SYNLETT, vol. 24, no. 14, 21 August 2013 (2013-08-21), pages 1785-1790, XP055210521, ISSN: 0936-5214, DOI: 10.1055/s-0033-1339487 cited in the application
- STÉPHANE LEBRUN ET AL: "Chiral Phase-Transfer-Catalyzed Intramolecular aza-Michael Reactions for the Asymmetric Synthesis of Isoindolinones", EUROPEAN JOURNAL OF ORGANIC CHEMISTRY, vol. 2015, no. 9, 5 March 2015 (2015-03-05), pages 1995-2004, XP055210513, ISSN: 1434-193X, DOI: 10.1002/ejoc.201403573
- DENG W ET AL: "Amino acid-mediated Goldberg reactions between amides and aryl iodides", TETRAHEDRON LET, ELSEVIER, AMSTERDAM, NL, vol. 45, no. 11, 8 March 2004 (2004-03-08) , pages 2311-2315, XP004491436, ISSN: 0040-4039, DOI: 10.1016/J.TETLET.2004.01.119

## Description

The present invention relates to the asymmetrical synthesis of heterocyclic compounds of pharmaceutical interest. In particular a series of enantioselective methods is described, allowing the synthesis of isoindolinones of following Formula **I**, into high yields and enantiomeric excesses >90% 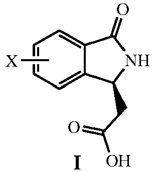 wherein the meaning of X is shown in the description.

### State of art

The importance and the widespread use of a skeleton with isoindolinone structure in several drugs both of natural and synthetic origin, in fact, has lead to a considerable interest in the synthesis thereof (Riedinger, C et. al. J. Am. Chem. Soc. 2008, 130, 16038; Couture, A. et al. Tetrahedron 2000, 56, 149). Several efforts were focused exactly towards the preparation of 3-substituted isoindolinones, considering the wide range of the biological activities thereof (Wrobel, J. et al. J. Med. Chem. 1992, 35, 4613; Belliotti et al. Bioorg. Med. Chem. Lett., 1998, 8, 1499). For example, important 3-substituted isoindolinones active on the central nervous system are JM-1232 (Nishiyama, T.; Chiba, S.; Yamada, Y. Eur. J. Pharmacol. 2008, 596, 56; Uemura, S. et al. Biochem. Biophys. Res. Commun. 2012, 418, 695) and DN-2327 (Hussein, Z. et al. Br. J. Clin. Pharmacol. 1993, 36, 357; Kondo, T.; Yoshida, K.; Yamamoto, M.; Tanayama, S. Arzneim. Forsch. 1996, 46, 11), agonists of receptor of benzodiazepine for treating anxiety, or the PD172939 derivative which, instead, is a powerful antagonist of the dopaminergic receptor D₄, useful in treating schizophrenia (Belliotti et al. Bioorg. Med. Chem. Lett., 1998, 8, 1499).

As the absolute configuration of the stereocenter is decisive to the purpose of the pharmacological activity (in most cases enantiomer S is the active one from a biological point of view (N. Kanamitsu et al., Chem. Pharm. Bull. 2007, 55, 1682 and EP1566378B1; Belliotti et al. Bioorg. Med. Chem. Lett., 1998, 8, 1499), the development of short, versatile and effective procedures for the enantioselective synthesis of these isoindolinones arouses great interest.

The production processes shown in literature are not at all satisfying due to the quite low yields and/or the not very high enantiomeric excesses. In the mentioned work, (N. Kanamitsu et al.Chem. Pharm. Bull. 2007, 55, 1682) the synthesis of enantiomers is carried out by means of resolution of the racemic mixture, by making to react the analogous racemic isoindolinone carboxylic acid of **I** (with X=cycloalkyl and bearing a substitute on the lactam ring), intermediate of JM1232, with phenyltilamine (R or S) to form the salt which is repeatedly crystallised by methanol/ethanol. The salt is then dissolved in water and washed with hydrochloric acid in order to obtain the enantiomerically enriched carboxylic acid but with extremely low yields (4%).

R. Sallio et al. (Synlett, 2013, 24, 1785) e S. Lebrun et al. (Eur. J. Org. Chem. 2015, 9, 1995) show the enantioselective preparation of *N,N*-disubstituted acetamido isoindolinones, mediated by the use of chiral catalysts with phase transfer in an intramolecular aza-Michael reaction of suitable benzamides, obtained starting from the tert-butyl ester of the 2-bromo benzoic acid and acrilamides by means of a catalysed palladium reaction. Such method has a series of disadvantages. The main disadvantage lies in the fact that the process shows a low versatility as the enantioselectivity strongly depends upon the used substrate and then even upon the used conditions: according to the used acrilamide, very different enantiomeric excesses are obtained. Furthermore, the proposed methods do not allow obtaining **I**, whereas the corresponding esters are obtained with poor selectivity as racemic mixture.

The isoindolinone called PD 172939 is synthesized as racemate, whereas the enantiomer (S) of PD 172939 is pharmacologically more active (Belliotti et al. Bioorg. Med. Chem. Lett., 1998, 8, 1499). (S)-PD172939 is obtained by means of resolution of the racemic mixture of PD172939 by using a chiral resolving agent, the malic acid, and by repeated crystallisation of the obtained salt. There are several disadvantages in this approach shown in literature. They relate to poor versatility, low yield (<10%) and poor enantioselective effectiveness. In fact, as one has to proceed with resolution of the racemic mixture of the synthesis' end product, very low yields are obtained only in the crystallisation process (<10%); the process low versatility is a considerable disadvantage as it allows to obtain one single chiral derivative at a time and it does not guarantee a structural diversification and high enantiomeric excesses; moreover, it is necessary using a chiral resolving agent in stechiometric quantities and additional purification steps; all these aspects have limited the applications of such derivatives.

Therefore, in the state of art the need was felt to have available effective methods allowing an enantioselective synthesis of precursors of above-mentioned molecules, identified in the isoindolinones by the structure **I** and **II,** and subsequently to transform them into the pharmacologically active compounds, identified by the general structures **III, IV, V** and **VI** by means of selective reactions which do not alter the enantiomeric purity. From a deep analysis of the literature it comes out that there are several methods allowing the synthesis of compounds **I** and **II** in racemic mixture, but there are no effective enantioselective procedures for obtaining them in enantioenriched way. Different compounds with structure **I,** such as (3-oxo-2,3-dihydro-1*H*-isoindol-1-il)acetic acid, (3-oxo-2,3-dihydro-1*H*-isoindol-1-yl)acetic acid), are commercially available as racemic mixtures even with very high costs, whereas the single enantiomers have never been described.

Thomas R. Belliotti et al "Isoindolinone enantiomers having affinity for the dopamine D4 receptor" Biorganic & Medicinal Chemistry Letters, vol. 8, no. 12, June 1998 (1998-06), pages 1499-1502, and US 6,087,364 describe synthetic methods for obtaining the racemic mixture of the compound I of the present invention, that is a 50/50 mixture of two enantiomers. However, the procedures described in the two mentioned documents are not applicable for the production of the single enantiomers.

Generally, for most part of the related chiral drugs, one of the enantiomers usually shows greater activity and/or less side effects than the respective racemic mixture or the other enantiomer. However, the need for effective methods for producing enantio-enriched drugs or intermediary thereof is not a trivial, but a crucial task for scalability, effective economic development of a production process of industrial interest. For the same reason, the same enantio-enriched intermediaries have an added value with respect to the racemic mixture if the usefulness thereof is demonstrated in the asymmetric synthesis of chiral drugs. When effective processes of asymmetrical synthesis are available, the chiral drugs can be produced with lower costs and with higher yields with respect to when the resolutions of racemes are used, processes having yields lower than 50% and which require additional purifications.

According to the present invention, the process for obtaining I is effective, easily scalable, selective and never described as such in the prior art. Furthermore, no racemization takes place during the reaction and the purification phases, a not obvious advantage for asymmetric syntheses of industrial interest. The compound I has never been described in know art as single enantiomer nor processes capable of separating it from the mixture of enantiomers were known. The present invention has further demonstrated the possibility of using it in a sequence of selective processes for the production of enantioenriched drugs. As a consequence, the compound I according to the present invention is not described as single enantiomer in the above-mentioned documents, nor the same describe methods which would allow the separation thereof in an obvious, efficient and industrially compatible way. Moreover EP 0529778 describes a series of 3-substituted isoindolinones the synthesis processes thereof cannot be connected to those described in the present invention, nor the process for producing I can be derived therefrom directly or indirectly.

Thomas R. Belliotti et al "Isoindolinone enantiomers having affinity for the dopamine D4 receptor" Biorganic & Medicinal Chemistry Letters, vol. 8, no. 12, June 1998 (1998-06), pages 1499-1502, describe processes applied on racemic mixtures which we have suitably modified to produce some of the enantiomerically enriched compounds subject of this invention.

US patent No. 6,087,364 describes compounds that are antagonists of dopamine D4 receptors, and the synthesis for preparing them.

US patent No. 4,244,966 describes derivatives of 1,3-dihydro-3-(2-hydroxyethyl)-2H-isoindol-1-one and the process for preparing them.

Now it has been surprisingly found that the isoindolinones of Formula **I** can be easily obtained with high yields and high enantiomeric purity and they can be used as starting compounds to obtain highly functionalized derivatives, pharmacologically active chiral products and derivatives thereof without racemization, with high yields and high enantiomeric excesses.

### Description of the invention

Therefore, the subject of the present invention is a process as defined in the claims. The subject of the present description are:
- a process for preparing compound of Formula **I** with enantiomeric purity (ee) > 90%
- processes for the synthesis of compounds of Formula **II** starting from the compound of Formula **I** and which keep unaltered the same enantiomeric purity with ee> 90%
- pharmacologically active compounds obtainable from such compounds
- uses of compound of Formula **I**

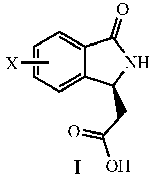

In the compounds of Formula **I,** according to the invention, the isoindolinone aromatic ring can be mono- or polysubstituted with a **X** group that may represent hydrogen, halogen, a C₁-C₁₀ alkyl group or a alkoxy C₁-C₁₀ group, nitro, phenyl.

In the synthesis of compounds of Formula **I** as starting materials 3-substituted isoindolinones of general Formula **A** or compounds of Formula **B** are used, both with enantiomeric purity (ee) > 90%, with R that may represent C₁-C₁₀ alkyl groups and X that may represent hydrogen, halogen, a C₁-C₁₀ alkyl group or a C₁-C₁₀ alkoxy group, nitro, phenyl. The compounds of Formula **A** can be obtained in both enantiomers according to the methods reported in RSC Advances, 2012, 2, 3592, whereas the compound **B** can be obtained by saponifying the compound **A** with a strong methanol base.

The advantages of the invention relate to the selective transformation of compounds of type **A** by means of decarboxylation reaction to give selectively the compounds **I** with high yields and without variation in enantiomeric excesses (Scheme S1). Alternatively, the compounds **I** can be prepared to start from the diacid **B** by saponifying the ester **A** with NaOH 2M in methanol and by subsequent decarboxylation under acidic conditions. Even in this case the compounds **I** are obtained without variation in the enantiomeric excesses.

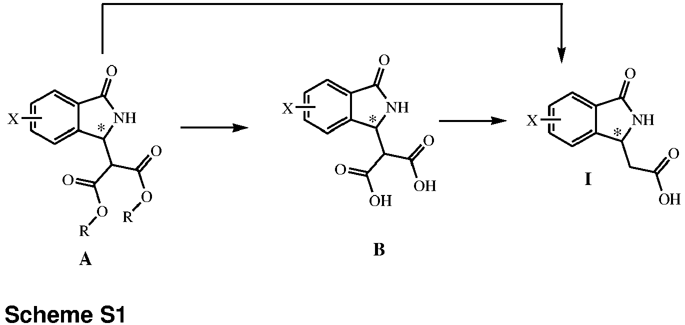

Compounds **C,** obtained by esterification from **I,** are conveniently transformed into compounds with structure **II** with high yields and high enantiomeric purity by means of reduction reaction advantageously performed with LiBH₄, lithium borohydride (Scheme S2), whereas other reducing agents such as NaBH₄ and LiAIH4 failed. Compounds I and **II** are conveniently transformed into compounds **III, IV, V** e **VI** variously functionalized in high yields and high enantiomeric purity. In particular selective methods are proposed leading to substituted compounds on amide nitrogen with Ar groups by means of arylation reactions catalysed by complexes of Cu(I), already known in literature for the versatility thereof but previously applied to racemic lactams (S. L. Buchwald, et al. J. Am. Chem. Soc. 2002, 124, 7421; A. Massa et al. Eur JOC, 2012, 5357); compounds bearing different amide substituents linked to the carboxylic acid, obtained with high yields and with enantiomeric purity (ee) > 90% by means of amidation reactions of **I;** or transformations of the derivative **II** leading to (S)-PD172939 and to structurally similar derivatives, with high yields and high enantiomeric excesses ee>90%.

In the structures **III, IV** and **VI,** Y represents N with R' an alkyl C₁-C₁₀, aloalkyl, aromatic or heteroaromatic group with 10 atoms in the ring, the aromatic or heteroaromatic rings could be unsubstituted or substituted with halogen, nitro, C₁-C₁₀ alkoxy, C₁-C₁₀ alkyl or C₁-C₁₀ aloalkyl.

All applied methods have the advantage of not causing even partial variations in the enantiomeric purity of the various starting products before and after the subject transformations. Such purity is analytically analysed by means of HPLC, high performance chromatography, on analytical column in chiral phase.

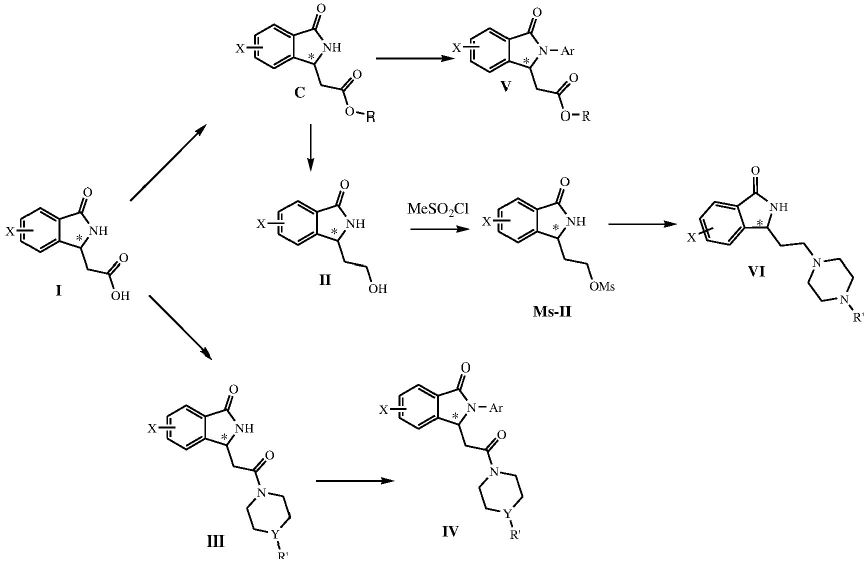

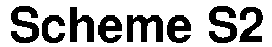

The term "alkyl" designates a C₁ - C₁₀ linear or branched chain. Examples are methyl, ethyl, isopropylic, butyl, pentyl and sec-butyl groups.

The term "alkoxy" designates C₁ - C₁₀ groups including metoxy, etoxy, tert-butoxy and isobutoxy, whereas the term "halogen" designates chlorine, fluorine, bromine and iodine.

All compounds of Formula **II, III, IV, V** and **VI** are chiral and they are of particular interest for the production of pharmacologically active compounds. They are obtained with high yields (85%) and with enantiomeric purity (ee) > 90%.

The compounds of Formula **I** - 2-(oxoisoindolin-3-yl) - acetic acids - can be obtained by means of selective transformation under acidic conditions of dialkyl 2-(1-oxoisoindolin-3-yl) malonate **A,** with ee> 90% implemented with HCI 6M for reaction time lower than 45 minutes, preferably 30 minutes, with temperatures >80°C, preferably >95°C.

By means of reducing chiral compounds of Formula **C** (preferably by means of LiBH₄ in THF under anhydrous conditions) compounds of Formula **II** with high yields (>85%) with high enantiomeric purity (ee>90%) can be obtained. The compounds of formula **III** with enantiomeric purity ee> 90% and yield > 85% can be obtained by means of the amidation of compounds of Formula **I** with enantiomeric purity (ee) > 90% with piperazine derivatives, carried out in presence of *N*-(3-dimethylaminopropyl)-N-ethylcarbodiimide. The compounds of Formula **II** can be made to react to obtain the compounds of Formula **VI** which keep the configurations of the compounds therefrom they have been obtained (Formula **I** and **II**), with high yields (>85%) and high enantiomeric purity (ee>90%).

The compounds of Formula **III** and **C** can be obtained directly starting from the compounds of Formula **I** with high yields and without variations in the enantiomeric excesses, whereas the compounds of Formula **IV** and **V** are obtained from **III** and **C** with high yields and with identical enantiomeric purity.

### Examples

### Hereinafter a series of examples is described, representing the various transformations, which do not have to be considered as limiting the present invention.

### Example 1

### SYNTHESIS OF 2-(1 oxoisoindolin-3-yl) MALONIC ACID (S)-B

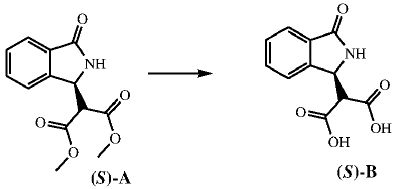

200 mg of (*S*)-**A** dimethyl 2-(1oxoisoindolin-3-yl) malonate (0.760mmol) compound with enantiomeric purity of 95% are solubilized in 4 mL of CH₂Cl₂/MeOH (9/1) and 2.5mL of NaOH 2M are added in MeOH. The reaction is left under electromagnetic stirring, at room temperature all night. The solvent at the rotavapor is moved away and one acidifies with HCI 2M until a pH=2. One extracts with Et₂O for three times, one anhydrifies on anhydrous sodium sulphate, one filters, one brings dry at the rotavapor by recovering a white solid (*S*)-**B** having a weight of 178 mg. Yield: 99%. P.f. 176°-177°C. [α]_{D} = - 6.3 (0.1 M MeOH). ESI (m/z): 236[M + H⁺].¹H NMR (300 MHz, D₂O) δ 7.72 (d, 1H, *J*= 6.87 Hz), 7.63-7.59 (m, 1H), 7.54-7.49 (m, 2H) 5.21 (d, 1H, J=8.55 Hz) 3.68 (d, 1H, *J*=8.55 Hz). ¹³C NMR (100 MHz, DMSO-d₆) δ 169.7, 169.2, 168.8, 145.8, 133.0, 131.5, 128.7, 124.0, 123.1, 55.8, 55.1. Elementary analysis for C₁₁H₉NO₅. Calculated: C, 56.17; H, 3.86; N, 5.96. Found: C, 56.25; H, 4.04; N, 6.12.

### EXAMPLE 2

### SYNTHESIS OF 2-(1oxoisoindolin-3-yl) ACETIC ACID (S)- I

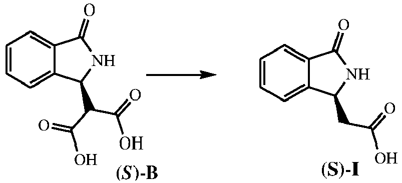

120 mg (0.51 mmol) of (*S*)-**B** diacid are suspended in HCI 6M (2 mL) and it is kept in reflux for 15 minutes. One extracts for three times with ethyl acetate, one anhydrifies on anhydrous sodium sulphate, one filters, one brings dry at the rotavapor by recovering (S)-**II** as a white solid having the weight of 94 mg. Yield: 97%. P.f.171°-172°C. ESI (m/z): 192 [M + H⁺]. [α]_{D}= -23 (c 1.0, MeOH). The enantiomeric excesses were determined after derivatization in the methylester according to the procedure described in example 4.

### EXAMPLE 3

### SYNTHESIS OF 2-(1oxoisoindolin-3-yl) ACETIC ACID (S)-I

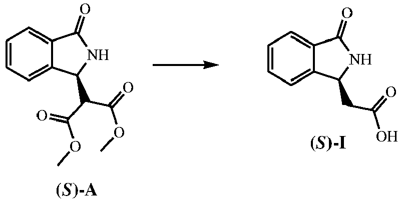

120 mg (0.46 mmol) of (*S*)-**A** dimethyl 2-(1-oxoisoindolin-3-il)malonate with enantiomeric purity del 95% are suspended in HCI 6N (2 mL) and the reaction is kept at reflux for 30 minutes. One extracts for three times with ethyl acetate. One anhydrifies on Na₂SO₄ and one filters on cotton-wool. One brings at the rotavapor, by recovering **(S)-I** as a whitish solid having the weight of 86 mg, purified by means of chromatography on silica gel, by using as eluent ethyl acetate. Yield:88% (77 mg). P.f.170°-171°C. ESI (m/z): 192 [M + H⁺]. [α]_{D}= -21 (c 1.0, MeOH). ¹HNMR (300 MHz, CD₃OD) δ: 7.76 (d, 1H, *J* =7. 5 Hz ), 7.65-7.53 (m, 2H), 7.51-7.48 (m, 1H), 5.09-4.99 (m, 1H), 2.97-2.89 (m, 1H), 2.68-2.48 (m, 1H).¹³CNMR (75 MHz, CD₃OD) δ: 172.6, 171.2, 146.8, 131.9, 131.3, 128.1, 122.9, 122.6, 53.4, 38.3. Elementary analysis for C₁₀H₉NO₃. Calculated: C, 62.82; H, 4.74; N, 7.33. Found: C, 62.62; H, 4.54; N, 6.99. The enantiomeric excesses were determined after derivatization in the methylester according to the procedure described in the example 4 or in the amides according to the procedure described in examples 12 and 13.

### EXAMPLE 4

### SYNTHESIS OF METHYL 2-(1oxoisoindolin-3-yl) ACETATE (S)-C

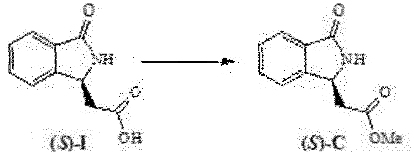

Two experiments are implemented separately by solubilizing 35 mg (0.18 mmol) of monoacid (*S*)-**I**, obtained from example 2 or from example 3, and 32 mg of K₂CO₃ (0.23 mmol) in DMF (1 mL) and after 20 minutes 18µL of iodomethane are added, by leaving the reaction under stirring all night. The solvent at the rotavapor is moved away and one purifies by means of chromatography on silica gel by using ethyl acetate, by recovering a white solid. Yield: 98% (35 mg). P.f. 145.6-147.7 °C; [α]_{D}= - 9.4 e -9.7 respectively (c 1.0, CHCl₃). MS (ESI): *m*/*z* = 206 [M + H⁺]. Elementary analysis for C₁₁H₁₁NO₃: Calculated C, 64.38; H, 5.40; N, 6.83. Found: C,64.52; H, 5.30; N, 6.68. The spectroscopic data are in agreement with what reported in literature for the racemic compounds (More et al., Synthesis, 2011, 3027). The enantiomeric excesses for both samples of 95% were determined by means of the column on chiral phase Chiralpack IA3, hexane-i-PrOH 8/2, 0.6 mL/min, λ= 254 nm t= 16.1 min and 20.3 min) by comparison with the racemic standard.

### EXAMPLE 5

### SYNTHESIS OF METHYL 2-(1oxoisoindolin-3-yl) ACETATE (R)-C

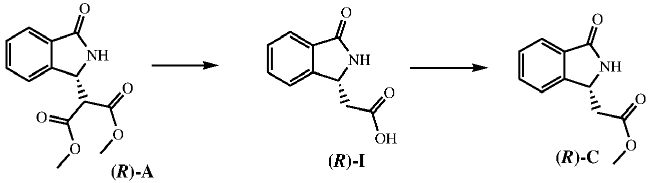

60 mg (0.228 mmol) of (*R*)-**A** dimethyl 2-(1-oxoisoindolin-3-il)malonate with enantiomeric purity del 95% are suspended in HCl 6N (1 mL) and the reaction is kept at reflux for 30 minutes. One extracts for three times with ethyl acetate. One anhydrifies on Na₂SO₄ and one filters on cotton-wool. One brings it at the rotavapor, by recovering (R)-**I** as a white solid having the weight of 43 mg, purified by means of chromatography on silica gel, by using as eluent ethyl acetate. Yield: 83% (36 mg). M.p171°-172°C. ESI (m/z): 192[M + H⁺].[α]_{D}= +21 (c 1.0, MeOH). Subsequently 32 mg (0.16 mmol) of monoacid (*R*)-**I**, and 32 mg of K₂CO₃ (0.23 mmol) in DMF (1 mL) and after 20 minutes 18µL of iodomethane are added, by leaving the reaction under stirring all night. The solvent at the rotavapor is moved away and one purifies by means of chromatography on silica gel by using ethyl acetate, by recovering a white solid. Yield: 98% (31mg), mp 146.6-148.7 °C; [α]_{D}= +10. The spectroscopic data are in agreement with what reported in literature for the racemic compounds (More et al., Synthesis, 2011, 3027). The enantiomeric excesses which resulted to be of 95% were determined by means of the column on chiral phase Chiralpack IA3 hexane-i-PrOH 8/2, 0.6 mL/min, λ= 254 nm t= 16.1 min and 20.3 min) by comparison with the racemic standard.

### EXAMPLE 6

### SYNTHESIS OF 2-(6-BROMO-1oxoisoindolin-3-yl) MALONIC ACID (S)-Br-B

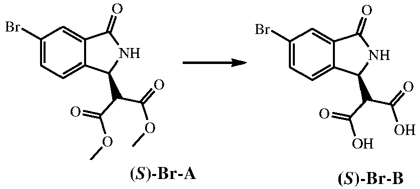

Analogously the derivative is synthesized starting from isoindolinone **(*S*)-Br-A** with ee 96%, according to the procedure described in the example 1. 160 mg of (*S*)-**Br-A** dimethyl 2-(6-bromo-1-oxoisoindolin-3-il)malonate (0.46 mmol) compound are solubilized in 3.6 mL of CH₂Cl₂/MeOH (9/1) and 2.6 mL of NaOH 2M are added in MeOH. The reaction is left under electromagnetic stirring, at room temperature all night. The solvent at the rotavapor is moved away and one acidifies with HCI 2M until a pH=2. One extracts with Et₂O for three times, one anhydrifies on anhydrous sodium sulphate, one filters, one brings dry at the rotavapor by recovering a white cerous solid having the weight of 130 mg.Yield: 91%. ESI (m/z): 313 [M + H⁺]. [α]_{D}= -41 (c 1.0, MeOH). Elementary analysis for C₁₁H₈BrNO₅. Calculated: C, 42.06; H, 2.57; N, 4.46. Found: C, 42.25; H, 2.24; N, 4.12. ¹HNMR (300 MHz, D₂O) δ 7.72 (d, 1H, *J*= 6.87 Hz), 7.63-7.59 (m, 1H), 7.54-7.49 (m, 2H) 5.21 (d, 1H, *J*=8.55 Hz) 3.68 (d, 1H, *J*=8.55 Hz). ¹³CNMR (75 MHz, D₂O) δ 172.6, 172.4, 171.2, 146.8, 131.9, 131.3, 128.1, 122.9, 122.6, 53.4, 52.4. The enantiomeric excesses were determined after derivatization in the methylester according to the procedure described in example 9.

### EXAMPLE 7

### SYNTHESIS OF 2-(6-BROMO-1oxoisoindolin-3-yl) ACETIC ACID (S)-Br-I

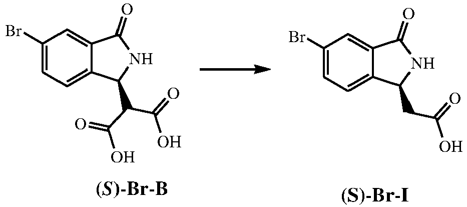

According to the procedure reported in example 2, 120 mg (0.38 mmol) of the diacid (S)-**Br-B** with ee 96% are suspended in HCl 6M (1.6 mL) and one keeps it at reflux for 30 minutes. One extracts for three times with ethyl acetate, one anhydrifies on anhydrous sodium sulphate, one filters, one brings dry at the rotavapor by recovering (S)-Br-**I** like a white ivory solid having the weight of 90 mg. Yield: 88%. The enantiomeric excesses were determined after derivatization in the methylester according to the procedure described in example 9.

### EXAMPLE 8

### SYNTHESIS OF 2-(6-BROMO-1oxoisoindolin-3-yl) ACETIC ACID (S)-Br-I

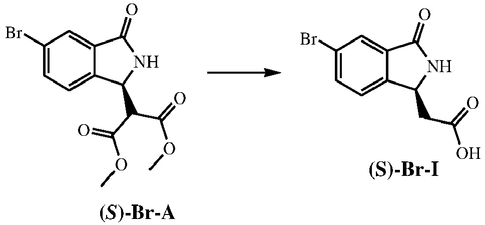

According to the procedure reported in example 3, 100 mg (0.292 mmol) of (*S*)-**Br A** dimethyl 2-(6-bromo-1-oxoisoindolin-3-il)malonate with ee 96% are suspended in HCl 6N (1.6 mL) and the reaction is kept at reflux for 45 minutes. One extracts for three times with ethyl acetate. One anhydrifies on Na₂SO₄ and one filters on cotton-wool. One brings it at the rotavapor, by recovering a white solid having the weight of 70 mg, purified by means of chromatography on silica gel, by using as eluent ethyl acetate. Yield:89%. P.f. 174°-175°C. ESI (m/z): 270 [M + H⁺]. [α]_{D}= -34 (c 1.0, MeOH). Elementary analysis for C₁₀H₈BrNO₃. Calculated: C, 44.47; H, 2.99; N, 5.19. Found: C, 44.32; H, 3.24; N, 4.98. ¹HNMR (250 MHz, CD₃OD) δ 7.87 (s, 1H), 7.74 (d, 1H, *J* =7.95 Hz), 7.54 (d, 1H, *J*=12 Hz), 5.20-5.18 (m, 1H), 2.97-2.84 (m, 1H), 2.65-2.55 (m, 1H).¹³CNMR (60 MHz, CD₃OD) δ172.6, 171.1, 146.1, 135.0, 126.2, 125.6, 124.9, 122.2, 53.5, 38.2. The enantiomeric excesses were determined after derivatization in the methylester according to the procedure described in example 9.

### EXAMPLE 9

### SYNTHESIS OF METHYL 2-(6-BROMO-1oxoisoindolin-3-yl) ACETATE (S)-Br-C

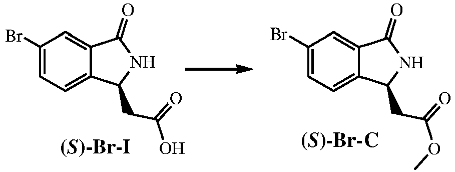

According to the procedure shown in example 4, two experiments are carried out separately by solubilizing 30 mg (0.11 mmol) of (S)-**Br-I** monoacid (obtained from example 7 or example 8) and 22 mg of K₂CO₃ (0.16 mmol) in DMF (1 mL) and after 20 minutes 15 µL of iodomethane are added, by leaving the reaction under stirring all night. The solvent at the rotavapor is moved away and one purifies by means of chromatography on silica gel by using ethyl acetate, by recovering a white solid having the weight of 28 mg. Yield: 90%. P.f. 123-124 °C; [α]_{D}= +1.6 (c 1.6, CHCl₃). MS (ESI): *m*/*z* = 283 [M + H⁺]. ¹HNMR (250 MHz, CDCl₃δ 7.98 (s, 1H), 7.70-7.54 (m 2H), 4.89 (d, 1H, *J*=10 Hz), 3.77 (s, 3H), 2.99 (d, 1H, *J*=10 Hz), 2.51-2.41 (m, 1H). ¹³CNMR (75 MHz, CDCl₃) δ171.3, 168.5, 144.2, 135.0, 132.1, 131.9, 127.3, 123.8, 53.1, 52.6, 38.9. Elementary analysis for C₁₁H₁₀BᵣNO₃: Calculated C, 46.50; H, 3.55; N, 4.93. Found: C, 46.52; H, 3.30; N, 4.68. The enantiomeric excesses for both samples which resulted to be of 96% were determined by means of the column on chiral phase Chiralpack IA3, hexane-i-PrOH8/2, 0.6 mL/min, λ= 254 nm t= 16.9 min and 24.0 min, by comparison with the racemic standard.

### EXAMPLE 10

### SYNTHESIS OF 3-(2-HYDROXYETHYL)ISOINDOLIN-1-ONE (S)-II

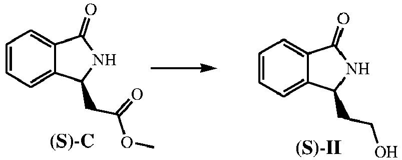

A solution of methyl ester (S)-**C** (60 mg, 0.292 mmol) in freshly distilled THF (1 mL) is kept under electromagnetic stirring in nitrogen atmosphere. A dilithium borohydride LiBH₄, 2M solution is made to drip slowly in THF (0.19 mL, 0.380 mmol) and the mixture is kept under stirring for 1h. The solvent at the rotavapor is moved away and one purifies on silica gel (ethyl acetate/methanol 95/5) by recovering a yellow oil having the weight of 44 mg. Yield: 86%. [α]_{D}=- 9.6 (c 0.25, CHCl₃). ESI (m/z): 178 [M + H⁺]. H, 4.34; N, 6.93. ¹HNMR (300 MHz, CDCl₃) δ 7.85 (d, 1H, *J*=7. 5 Hz), 7.57-7.43 (m, 3H), 4.75 (d app, 1H, J =6.1 Hz), 3.997-3.93 (m, 2H), 2.80 (br s, 1H), 2.28-2.24 (m, 1H), 1.73 (m, 1H). ¹³CNMR (60 MHz, CDCl₃) δ 170.8, 147.7, 132.1, 131.9, 128.4, 124.1, 122.6, 61.0, 56.2, 37.2. Elementary analysis for C₁₀H₁₁NO₂. Calculated: C, 67.78; H, 6.26; N, 7.90. Found: C, 67.48; H, 6.46; N, 7.87. The enantiomeric excesses which resulted to be of 95% were determined by means of the column on chiral phase Chiralpack IE3, hexane-i-PrOH 8/2, 0.6 mL/min, λ= 254 nm t=30.4 min and 34.4 min., by comparison with the racemic standard.

### EXAMPLE 11

### SYNTHESIS OF THE DERIVATIVE (S)-PD172939

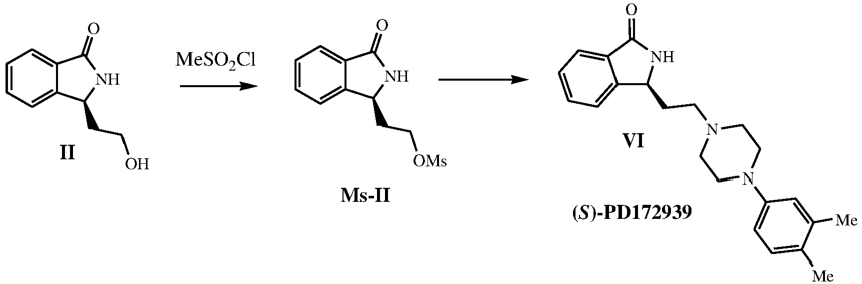

### STEP 1: 2-(1oxoisoindolin-3-yl)ethyl METHANESULPHONATE MS-II

Methanesulfonyl chloride (64 mg, 0.56 mmol, 48 µL) is added slowly to a solution of alcohol **II** (80 mg, 0.45mmol) and Et₃N (72 mg, 0.72 mmol, 96 µL) in CH₂Cl₂ and the reaction mixture is kept under electromagnetic stirring for 2h at room temperature under nitrogen stream. The solvent is moved away at reduced pressure and the residue purified on silica gel (ethyl acetate) to give a white solid. Yield: 88% (105 mg). P.f. 131-133 °C. ee 95%. [a]_{D}=- 33.6 (c 0.5, CHCl₃). ESI (m/z): 256 [M + H⁺].¹HNMR (400 MHz, CDCl₃) δ 7.83 (d, 1H, *J*= 7. 3 Hz), 7.63-7.53 (m, 1H), 7.51-7.47 (m, 3H), 4.83 (br s 1H), 4.42-4.41 (m, 2H), 2.53 (s, 3H), 2.51-2.49 (m, 1H), 2.00-1.97 (m 1H). ¹³CNMR (100 MHz, CDCl₃) δ 171.0, 146.4, 132.3, 128.6, 124.0, 122.5, 66.6, 53.6, 37.4, 34.2. Elementary analysis for C₁₁H₁₃NO₄S. Calculated: C, 51.75; H, 5.13; N, 5.49. Found: C, 51.58; H, 5.36; N, 5.67.

### STEP 2: 3-(2-(4-(3,4-DIMETHYLPHENYL)PIPERAZIN-1-IL)ETHYL)ISOINDOLIN-1-ONE (S)-PD 172938 VI.

Under nitrogen stream la 1-(3,4 dimethylphenyl)piperazine (53 mg, 0.28mmol) is added to a solution of mesilate Ms-**II** (60 mg, 0.23mmol) and Et₃N (72 mg, 0.72 mmol, 96 µL) in CH₂Cl₂ and the reaction mixture is kept under electromagnetic stirring at temperature of 50 °C all night. The solvent is moved away at reduced pressure and the residue purified on silica gel (Ethyl acetate/Methanol 90/10) to give a cerous white solid. Yield: 83% (105 mg). ee 95%. [a]_{D}=- 17 (c 0.75, CHCl₃). ESI (m/z): 350 [M + H⁺]. ¹HNMR (400 MHz, CDCl₃) δ 7.85 (d, 1H, *J* = 7.2 Hz ), 7.57-7.42 (m, 4H), 7.02 (d, 1H, *J* = 8.1 Hz), 6.75-6.68 (m, 1 H + NH), 4.63 (d app, 1H, *J* = 9.3 Hz), 3.20-3.16 (m, 4H), 2.75-2.54 (m, 6H), 2.23-2.18 (s + m, 7H), 1.80-1.72 (m, 1 H). ¹³CNMR (100 MHz, CDCl₃) δ 170.7, 170.3, 149.5, 149.4, 147.5, 137.1, 132.0, 131.7, 130.2, 128.3, 123.8, 122.3, 118.3, 114.0, 57.1, 56.5, 53.4, 49.8, 31.3, 20.2, 18.8. Elementary analysis for C₂₂H₂₇N₃O₂. Calculated: C, 75.61; H, 7.79; N, 12.02. Found: C, 75.48; H, 7.46; N, 11.87.

### EXAMPLE 12

### METHYL 2-[1oxo-2-(pyridin-2-yl)-isoindolin-3-yl] acetate (S)-V

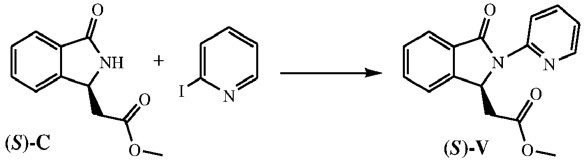

Cul (7 mg, 0.04mmol) and K₃PO₄ (74 mg, 0.35 mmol) are put in a Schlenk tube and three vacuum-nitrogen cycles are performed. In a nitrogen atmosphere *N,N-*dimethylethylenediamine (6 mg, 8.5 µl, 0.07 mmol), 2-iodo-pyridine (42 mg, 22 µl, 0.21 mmol) and chiral ester (S)-**C** (36 mg, 0.18 mmol) dissolved in dioxane (2 mL), are added in the order. The reaction mixture is kept under electromagnetic stirring at 80°C for 24 h. The obtained suspension having a brownish colour is cooled at room temperature and after solvent removal, purified by means of chromatography on silica gel (Acetate 7/Hexan 3), by recovering a white solid having the weight of 40 mg. Yield: 80%. [a]_{D}=+ 4.6 (c 1.6, CHCl₃). ESI (m/z): 283[M + H⁺]. Elementary analysis for C₁₆H₁₄N₂O₃. Calculated: C, 68.07; H, 5.00; N, 9.92. Found: C, 68.38; H, 5.16; N, 9.87. The spectroscopic data are in agreement with what reported in literature for the racemic mixture (Petronzi et al., Eur JOC, 2012, 5357). The enantiomeric excesses which resulted to be of 95% were determined by means of the column on chiral phase Chiralpack IA3 column, hexane-i-PrOH 8/2, 0.6 mL/min, λ= 254 nm t=12.4 min e 13.4 min., by comparison with the racemic standard.

### EXAMPLE 13

### 3-(2-OXO-2-(4-(4-METHYLPHENYL)PIPERAZINE-1-IL)ETHYL) ISOINDOLIN-1 - ONE (S)-IIIa

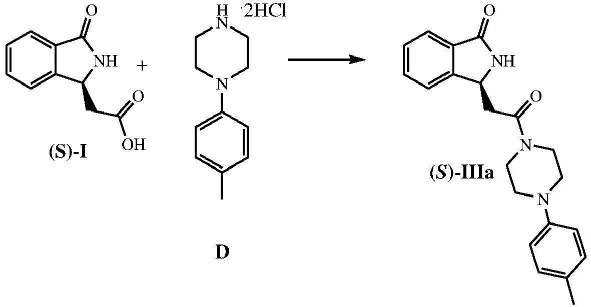

A solution containing monoacid (S)-**I** (60 mg, 0.32mmol), 1-tolilpiperazine dihydrochloride **D** (80mg, 0.32mmol), *N*-(3-dimethylaminopropyl)-*N*'-ethylcarbodiimide hydrochloride (60 mg, 0.32mmol), il 1-hydroxybenzotriazole (42 mg, 0.32mmol) and triethylamine (110 mg, 152 µl, 1.1 mmol) in tetrahydrofuran (4 ml) is kept under electromagnetic stirring at 25°C for 16h. The reaction mixture is concentrated under pressure and the residue recovered with chloroform is washed with water, dried on anhydrous sodium sulfate and purified by means of chromatography on silica gel (CHCl₃/MeOH 95/5). Yellow oil. Yield: 88% (96 mg). [α]_{D}= -57 (c 1.0, CHCl₃). MS (ESI): *m*/*z* 350 [M + H⁺].¹HNMR (250 MHz, CDCl₃) δ: 8.06 (d,1H, *J*= 7 Hz) 7.76-7.61 (m, 3H) 7.60 (s, 1H) 7.27(d, 2H, *J*= 8.3 Hz) 7.02 (d, 2H, *J*= 8.4Hz) 5.24 (d, 1H *J*= 10Hz), 4.04-4.00 (m, 2H), 3.76-3.72 (m, 2H), 3.33-3.24 (m, 5H), 2.71-2.60 (m, 1H), 2.46 (s, 3H).¹³CNMR(60 MHz, CDCl₃) δ: 170.3, 168.8, 148.7, 146.6, 132.2, 132.1, 130.7, 130.0, 128.8, 124.3, 122.5, 117.3, 53.6, 50.4, 50.1, 45.5, 41.9, 39.1, 20.6. Elementary analysis for C₂₁H₂₃N₃O₂. Calculated: C, 72.18; H,6.63; N, 12.03. Found: C, 71.97; H, 6.42; N, 11.73. The enantiomeric excesses which resulted to be of 95% were determined by means of the column on chiral phase Chiralpack IE3, hexane-i-PrOH 8/2, 0.6 mL/min, λ= 254 nm t = 33.4 min and 37.5 min, by comparison with the racemic standard.

### EXAMPLE 14

### 3-(2-OXO-2-(4-(4-FLUOROPHENYL)PIPERAZINE-1-IL)ETHYL) ISOINDOLIN-1-ONE (S)-IIIb

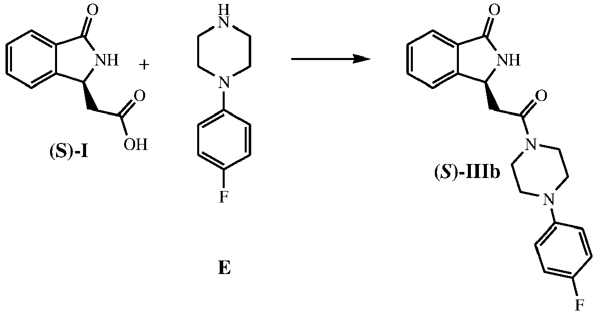

According to the procedure shown in example 12, a solution including the monoacid (*S*)-**I** (60 mg, 0.32mmol), 1-fluoropiperazine **E** (56 mg, 0.32mmol), *N*-(3-dimethylaminopropyl)-N'-ethylcarbodiimide hydrochloride (60 mg, 0.32mmol), 1-hydroxybenzotriazole (42 mg, 0.32mmol) and triethylamine (96µl, 0.70 mmol) in tetrahydrofuran (4 ml) is kept under electromagnetic stirring at 25°C for 16 h. The reaction mixture is concentrated under pressure and the residue recovered with chloroform is washed with water, dried on anhydrous sodium sulphate and purified by means of chromatography on silica gel (CHCl₃/MeOH 95/5). Yellow oil. Yield: 86% (96 mg). [α]_{D}= -54 (c 1.0, CHCl₃).MS(ESI): *m*/*z* = 376 [M + Na⁺]. C₂₀H₂₀FN₃O₂Calculated C, 67.97; H, 5.70; N, 11.89. Found C, 67.85; H, 5.60; N, 11.80. The spectroscopic data are in agreement with what reported in literature for the racemic compound (Petronzi et al., Eur JOC, 2012, 5357). The enantiomeric excesses which resulted to be of 95% were determined by means of the column on chiral phase Chiralpack IA3 column, hexane-i-PrOH 8/2, 0.6 mL/min, λ= 254 nm t = 40.0 min and 45.9 min., by comparison with the racemic standard.

### EXAMPLE 15

### 2-(5-NITROPIRIDIN-2-IL)-3-(2-OXO-2-(4-(4-METHYLPHENYL)PIPERAZIN-1-IL)ETHYL)ISOINDOLlN-1-ONE (S)-IV

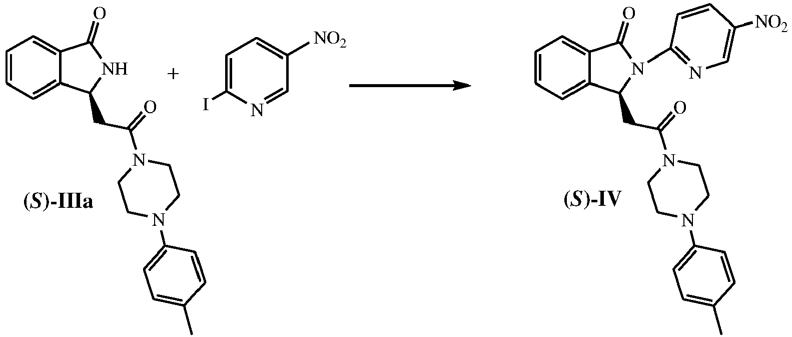

According to the procedure shown in example 10, Cul (6.6 mg, 0.035mmol) and K₃PO₄ (74 mg, 0.35mmol) are put in a Schlenk tube and three vacuum-nitrogen cycles are performed. In nitrogen atmosphere *N,N*-dimethylethylenediamine (6 mg, 8 µl, 0.070mmol), 2-iodo-5-nitropyridine (54 mg, 0.21 mmol) and amide (*S*)-**IIIa** (64 mg, 0.18 mmol) dissolved in dioxane (2 mL) are added in the order. The reaction mixture is kept under electromagnetic stirring at 80°C for 24 h. The obtained suspension having a brownish colour is cooled down at room temperature and after solvent removal purified by means of chromatography on silica gel (Acetate 3/Hexane 7), by recovering a yellow solid having the weight of 70 mg. Yield: 84%. [α]_{D}= + 45 (c 0.1, CHCl₃). P.f. 188°-189°C. MS (ESI): *m*/*z*= 472.51 [M + H⁺].¹HNMR (250 MHz, CDCl₃) δ 9.28 (s, 1H), 8.88 (d, 1H, *J*= 9.3 Hz), 8.53 (d, 1H, *J*= 9.3 Hz), 7.77-7.50 (m, 4H), 7.07 (d, 2H, *J*= 8 Hz), 6.81 (d, 2H, *J*= 8 Hz), 6.17 (d, 1H *J*= 9.55 Hz), 3.85-3.84 (m, 2H), 3.55-3.51 (m, 3H), 3.16-3.00 (m, 4H), 2.60-2.49 (m, 1H), 2.26 (s, 3H). ¹³CNMR (60 MHz, CDCl₃) δ 168.3, 168.2, 155.0, 148.8, 146.2, 144.6, 140.2, 134.2, 133.6, 130.6, 130.0, 129.2, 124.8, 124.3, 117.2, 114.3, 57.9, 50.4, 50.2, 45.7, 42.0, 37.5, 20.6. Elementary analysis for C₂₆H₂₅N₅O₄. Calculated: C, 66.23; H, 5.34; N, 14.85. Found: C, 65.91; H, 5.19; N, 14.83. The enantiomeric excesses which resulted to be of 95% were determined by means of the column on chiral phase Chiralpack IA3 column, hexane/i-PrOH 8/2, 0.6 mL/min, λ= 254 nm t = 30.02 min and 35.8 min, by comparison with the racemic standard.

## Claims

1. A process for the preparation of a compound of general formula I or its salts with enantiomeric purity ee>90% 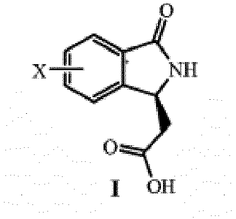 wherein
the isoindolinone aromatic ring can be mono- or polysubstituted with X that may represent H, halogen, an alkyl C₁-C₁₀ group or an alkoxy C₁-C₁₀ group, nitro, phenyl; said process comprising a decarboxylation process of formula A or of the diacid B, both with enantiomeric purity ee>90%
performed under acidic conditions, temperature >95°C and reaction time of less than 30 minutes
wherein R is an alkyl C₁-C₁₀ group according to the following Scheme S1:

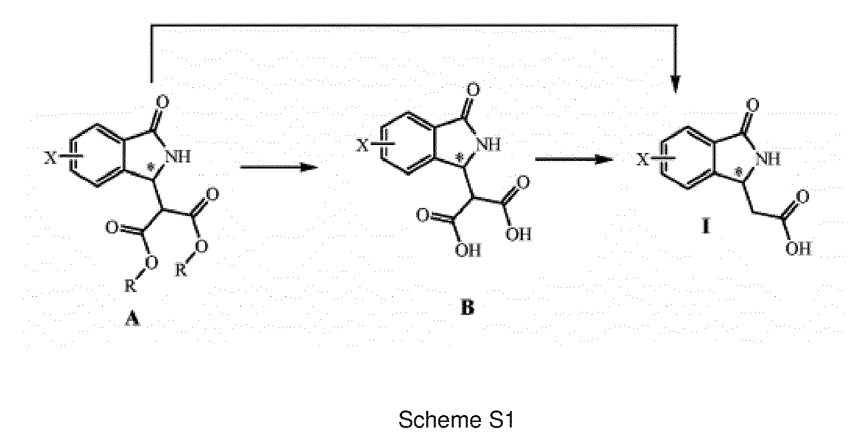

2. A process according to claim 1 further comprising the steps of producing the compounds of Formula C, II, V and VI as in the following Scheme 2 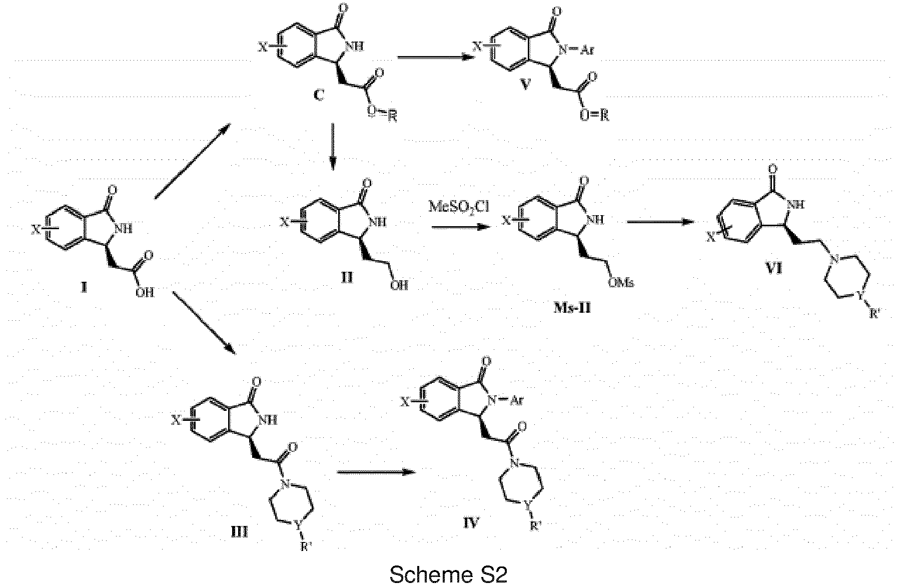 wherein R and X are as defined in claim 1
said process comprising an esterification reaction of compound of Formula I prepared as in claim 1, said esterification being carried out reacting primary alkyl halides in presence of K₂CO₃ to yield a compound of formula C; further comprising a reduction of said compound of Formula C according to Scheme S2 to yield a compound of Formula II with yields > 85% and ee>90%;
further comprising the reaction of said compound of Formula II according to Scheme S2 with methanesulfonyl chloride (MeSO₂Cl) in the presence of triethylamine and subsequent reaction of the obtained compounds Ms-II with secondary amines, carried out at 50°C under anhydrous conditions wherein Y is N and R', a C₁-C₁₀ alkyl, haloalkyl, aryl or heteroaryl group with 6 or 10 atoms in the ring, the aromatic or heteroaromatic rings can be unsubstituted or substituted with halogen, nitro, C₁-C₁₀ alkoxy, C₁-C₁₀ alkyl or C₁-C₁₀ haloalkyl, to yield a compound of Formula VI with ee > 90% and yield > 85%;
further comprising an arylation reaction of said compound of Formula C with aryl halides of Formula ArX, in the presence of Cul as catalyst, K₃PO₄ and *N,N-*dimethylethylenediamine in dioxane at T>75°C, wherein Ar is an aromatic or heteroaromatic ring with 6 or 10 atoms, unsubstituted or substituted with halogen, nitro, alkyl C₁-C₁₀, cycloalkyl C₁-C₁₀, alkoxy C₁-C₁₀, to yield a compound of Formula V as shown in Scheme S2.

3. The process according to claim 2, wherein said reduction reaction of said compound of Formula C according to Scheme S2 to yield a compound of Formula II is carried out with LiBH₄ (lithium borohydride).

4. A process according to claim 1 further comprising the steps of producing a compound of Formula III and IV as in Scheme S2 of claim 2 with ee> 90% and yield > 85% comprising the amidation of compounds of Formula I with ee> 90% prepared as in claim 1 carried out in the presence of cyclic secondary amines derived from piperazine, and in the presence of *N*-(3-dimethylaminopropyl)-*N*'-ethylcarbodiimide, wherein X has the meaning indicated in claim 1, Y is N and R', a C₁-C₁₀ alkyl, haloalkyl, aryl or heteroaryl group with 6 or 10 atoms in the ring, the aromatic or heteroaromatic rings can be unsubstituted or substituted with halogen, nitro, C₁-C₁₀ alkoxy, C₁-C₁₀ alkyl or C₁-C₁₀ haloalkyl to yield a compound of formula III according to Scheme S2 of claim 2,
further comprising an arylation reaction of compounds of Formula III with aryl halides ArX, in the presence of Cul as catalyst, K₃PO₄ and *N,N-*dimethylethylenediamine in dioxane at T>75°C, wherein Ar is an aromatic or heteroaromatic ring with 6 or 10 atoms of ring, unsubstituted or substituted, with halogen, nitro, alkyl C₁-C₁₀, cycloalkyl C₁-C₁₀, alkoxy C₁-C₁₀, to yield a compound of Formula IV as shown in Scheme S2 of claim 2 with ee> 90% and yield > 85%.

## Patentansprüche

1. Verfahren für die Herstellung einer Zusammensetzung der allgemeinen Formel I oder deren Salze mit Enantiomerenreinheit ee > 90 % 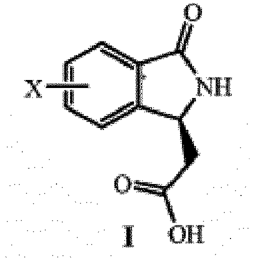
wobei der aromatische Isoindolinonring mit mono- oder polysubstituiert sein kann mit X, das H, Halogen, eine C₁-C₁₀-Alkylgruppe oder eine C₁-C₁₀-Alkoxygruppe, Nitro, Phenyl repräsentieren kann;
wobei das Verfahren einen Decarboxylierungsprozess einer Formel A oder der Disäure B enthält, beide mit einer Enantiomerenreinheit ee > 90 %,
ausgeführt unter sauren Bedingungen, bei einer Temperatur von > 95 °C und für einen Reaktionszeitraum von weniger als 30 Minuten,
wobei R eine C₁-C₁₀-Alkylgruppe gemäß dem folgenden Schema S1 ist:

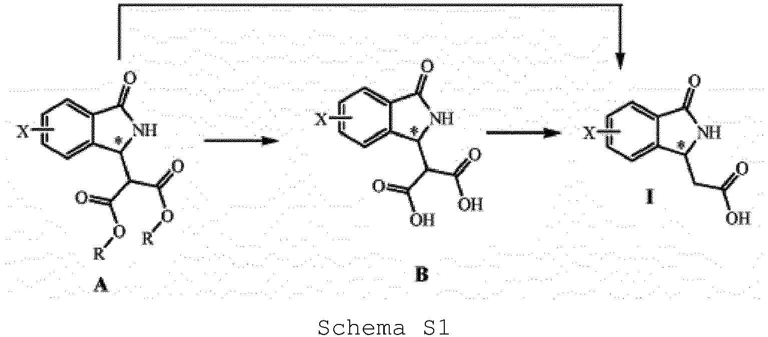

2. Verfahren nach Anspruch 1, wobei ferner enthalten sind die Schritte des Herstellens der Zusammensetzungen der Formeln C, II V und VI, wie in dem folgenden Schema 2 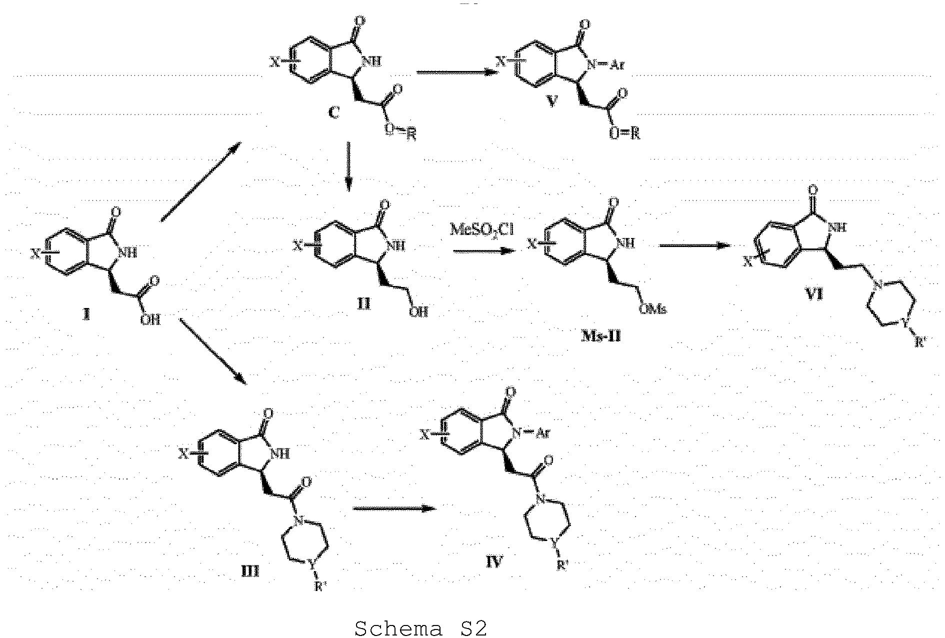
wobei R und X wie im Anspruch 1 definiert sind,
wobei der Prozess eine Veresterungsreaktion der Zusammensetzung der Formel I, durchgeführt wie im Anspruch 1, enthält, wobei die Veresterung ausgeführt wird unter primärem Reagieren von Alkylhalogeniden unter Gegenwart von K₂C0₃, um eine Zusammensetzung der Formel C zu erhalten; wobei ferner enthalten ist eine Reduktion der Zusammensetzung der Formel C gemäß dem Schema S2, um eine Zusammensetzung der Formel II zu erhalten, mit Erträgen > 85 % und ee > 90 %; wobei ferner enthalten ist, die Reaktion der Zusammensetzung der Formel II gemäß dem Schema S2 mit Methansulfonylchlorid (MeSO₂Cl) in Gegenwart von Triethylamin und anschließende Reaktion der erhaltenen Zusammensetzung Ms-II mit sekundären Aminen, durchgeführt bei 50 °C unter wasserfreien Bedingungen, wobei Y gleich N ist und R' ein C₁-C₁₀-Alkyl-, Halogenalkyl-, aromatische oder heteroaromatische Gruppe mit 6 oder 10 Atomen im Ring ist, wobei die aromatischen oder heteroaromatischen Ringe unsubstituiert oder mit Halogen, Nitro, C₁-C₁₀-Alkoxy, C₁-C₁₀-Alkyl oder C₁-C₁₀-Halogenalkyl substituiert sein können, um eine Zusammensetzung der Formel VI mit ee > 90 % und Ertrag > 85 % zu erhalten;
wobei ferner enthalten ist eine Arylierungsreaktion von Zusammensetzungen der Formel C mit Arylhalogeniden der Formel ArX, in Gegenwart von CuI als Katalysator, K₃PO₄ und *N,N-*Dimethylethylendiamin in Dioxan bei T > 75 °C, wobei Ar ein aromatischer oder heteroaromatischer Ring mit 6 oder 10 Atomen ist, unsubstituiert oder substituiert mit Halogen, Nitro, C₁-C₁₀-Alkyl, C₁-C₁₀-Zykloalkyl, C₁-C₁₀-Alkoxy, um eine Zusammensetzung der Formel V zu erhalten, wie im Schema S2 gezeigt ist.

3. Verfahren nach Anspruch 2, wobei die Reduktionsreaktion der Zusammensetzung der Formel C gemäß dem Schema S2, um eine Zusammensetzung der Formel II zu erhalten, mit LiBH₄ (Lithiumborhydrid) ausgeführt wird.

4. Verfahren nach Anspruch 1, wobei ferner die Schritte des Herstellens einer Zusammensetzung der Formeln III und IV wie im Schema S2 des Anspruchs 2 enthalten sind, mit ee > 90 % und Ertrag > 85 %, wobei enthalten ist die Amidierung von Zusammensetzungen der Formel I mit ee > 90 %, hergestellt wie im Anspruch 1, ausgeführt unter Gegenwart von zyklischen sekundären Aminen, die von Piperazin erhalten wurden, und in der Gegenwart von N-(3-Dimethylaminopropyl)-N-Ethylcarbodiimid,
wobei X die in Anspruch 1 angegebene Bedeutung hat, Y gleich N ist und R' eine C₁-C₁₀-Alkyl-, Halogenalkyl-, aromatische oder heteroaromatische Gruppe mit 6 oder 10 Atomen im Ring ist, wobei die die aromatischen oder heteroaromatischen Ringe unsubstituiert oder substituiert mit Halogen, Nitro, C₁-C₁₀-Alkoxy, C₁-C₁₀-Alkyl oder C₁-C₁₀-Halogenalkyl sein können, um eien Zusammensetzung der Formel III gemäß Schema S2 von Anspruch 2 zu erhalten
wobei ferner enthalten ist eine Arylierungsreaktion von Verbindungen der Formel III mit Arylhalogeniden der Formel ArX in Gegenwart von CuI als Katalysator, K₃PO₄ und *N,N-*Dimethylethylendiamin in Dioxan bei T > 75 °C, wobei Ar ein aromatischer oder heteroaromatischer Ring mit 6 oder 10 Ringatomen ist, unsubstituiert oder substituiert mit Halogen, Nitro, C₁-C₁₀-Alkyl, C₁-C₁₀-Zykloalkyl, C₁-C₁₀-Alkoxy, um eine Zusammensetzung der Formel IV, wie im Schema S2 von Anspruch 2 gezeigt ist, mit ee > 90 % und Ertrag > 85 % zu erhalten.

## Revendications

1. Un procédé pour la préparation d'un composé de formule générale I ou de ses sels avec une pureté énantiomérique ee>90%. 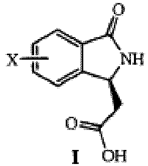 où
l'anneau aromatique isoindolinone peut être mono- ou poly-substitué par X qui peut représenter H, un halogène, un groupe alkyle en C₁-C₁₀ ou un groupe alcoxy en C₁-C₁₀, un nitro, un phényle ; ledit procédé comprenant un procédé de décarboxylation de la formule A ou du diacide B, tous deux avec une pureté énantiomérique ee>90% réalisé dans des conditions acides, à une température >95°C et avec un temps de réaction inférieur à 30 minutes, où R est un groupe alkyle en C₁-C₁₀ selon le Schéma S1 suivant :

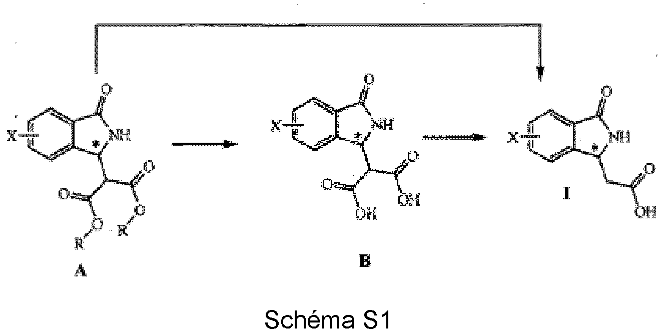

2. Un procédé selon la revendication 1 comprenant en outre les étapes de production des composés de Formules C, Il, V et VI comme dans le Schéma S2 suivant : 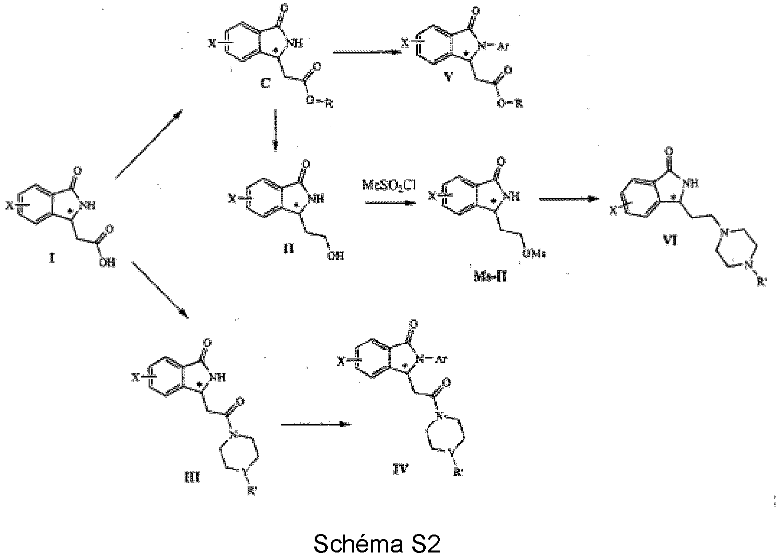
où R et X sont tels que définis dans la revendication 1 ledit procédé comprenant une réaction d'estérification du composé de Formule I préparé selon la revendication 1, ladite estérification étant effectuée en faisant réagir des halogénures d'alkyle primaires en présence de K₂CO₃ pour donner un composé de Formule C ; comprenant en outre une réduction dudit composé de Formule C selon le Schéma S2 pour donner un composé de Formule Il avec des rendements >85 % et un ee>90 % ;
comprenant en outre la réaction dudit composé de Formule II selon le Schéma S2 avec du chlorure de méthane-sulfonyle (MeSO₂Cl) en présence de tri-éthylamine et la réaction subséquente des composés obtenus Ms-II avec des amines secondaires effectuée à 50°C dans des conditions anhydres où Y est N et R' est un groupe alkyle, halo-alkyle,
aryle ou hétéro-aryle en C₁-C₁₀ à 6 ou 10 atomes dans l'anneau, les anneaux aromatiques ou hétéro-aromatiques peuvent être non substitués ou substitués par un halogène, un nitro, un alkoxy en C₁-C₁₀, un alkyle en C₁-C₁₀ ou un halo-alkyle en C₁-C₁₀, pour donner un composé de Formule VI avec un ee >90% et un rendement >85% ;
comprenant en outre une réaction d'arylation dudit composé de Formule C avec des halogénures d'aryle de Formule ArX, en présence de Cul en tant que catalyseur, de K₃PO₄ et de N,N-diméthyl-éthylène-diamine dans du dioxane à T>75°C, où Ar est un anneau aromatique ou hétéro-aromatique à 6 ou 10 atomes, non substitué ou substitué par un halogène, un nitro, un alkyle en C₁-C₁₀, un cyclo-alkyle en C₁-C₁₀, un alcoxy en C₁-C₁₀, pour donner un composé de Formule V comme indiqué dans le Schéma S2.

3. Le procédé selon la revendication 2, dans lequel ladite réaction de réduction dudit composé de Formule C selon le Schéma S2 pour donner un composé de Formule II est effectuée avec du LiBH₄ (borohydrure de Lithium).

4. Le procédé selon la revendication 1 comprenant en outre les étapes de production d'un composé de Formules III et IV comme dans le Schéma S2 de la revendication 2 avec un ee>90% et un rendement >85% comprenant l'amidation de composés de Formule I avec un ee>90% préparés comme dans la revendication 1 en présence d'amines secondaires cycliques dérivées de la pipérazine, et en présence de N-(3-diméthylaminopropyle)-N'-éthylcarbodiimide,
où X a la signification indiquée dans la revendication 1, Y est N et R' est un groupe alkyle, halo-alkyle, aryle ou hétéro-aryle en C₁-C₁₀ avec 6 ou 10 atomes dans l'anneau, les anneaux aromatiques ou hétéro-aromatiques peuvent être non substitués ou substitués avec un halogène, un nitro, un alcoxy en C₁-C₁₀, un alkyle en C₁-C₁₀ ou un halo-alkyle en C₁-C₁₀ pour donner un composé de Formule III selon le Schéma S2 de la revendication 2,
comprenant en outre une réaction d'arylation de composés de Formule III avec des halogénures d'aryle ArX, en présence de Cul comme catalyseur, de K₃PO₄ et de N,N-diméthyléthylènediamine dans du dioxane à T>75°C, où Ar est un anneau aromatique ou hétéro-aromatique à 6 ou 10 atomes, non substitué ou substitué avec un halogène, un nitro, un alkyle en C₁-C₁₀, un cyclo-alkyle en C₁-C₁₀, un alcoxy en C₁-C₁₀, pour donner un composé de Formule IV comme dans le Schéma S2 de la revendication 2 avec un ee>90% et un rendement >85%.
